# EUROPEAN PATENT APPLICATION

(11) **EP 3 031 419 A1**
(43) Date of publication of application: **15.06.2016**
(21) Application number: 14819727.0
(22) Date of filing: 04.07.2014
(51) Int. Cl.: A61B 18/12, A45D 26/00

(54) **DEPILATORY DEVICE CONTROL METHOD AND DEPILATORY DEVICE**

(30) Priority: 04.07.2013 CN 201310279972; 01.07.2014 CN 201410312038
(71) Applicant: 405 Limited Company (Shenzhen), Guangdong (CN)
(72) Inventor: YOKO, Oguchi, Yokohama-shi Kanagawa (JP)
(74) Representative: Zaboliene, Reda
(86) International application number: PCT/CN2014/081698
(87) International publication number: WO 2015/000443

(57) **Abstract**

A control method for an electrical hair removal device, the electrical hair removal device including a heat generator having a heating wire formed thereon, including the steps of step S11: starting the electrical hair removal device and letting it go into a standby state; step S12: placing the heat generator on an hair removal needed area; and step S13: electrifying the heating wire to a predetermined temperature, for realizing hair removal with a constant heating temperature. An electrical hair removal device includes a main body and a cover for accommodating the main body. The main body has a heat generator having a heating wire thereon, a supporting element and a printed circuit board (PCB). The heating wire is electrically connected to the PCB, which is electrified and heated to a predetermined temperature, for realizing hair removal with a constant heating temperature.

## Description

### RELATED APPLICATIONS

This application is a PCT National Stage of PCT Patent Application No. PCT/CN2014/081698, filed July 4, 2014, and claims benefit to Chinese Patent Application No. CN201310279972.4, filed July 4, 2013, and Chinese Patent Application No. CN201410312038.2, filed July 1, 2014.

The above applications and all patents, patent applications, articles, books, specifications, other publications, documents, and things referenced herein are hereby incorporated herein in their entirety for all purposes. To the extent of any inconsistency or conflict in the definition or use of a term between any of the incorporated publications, documents, or things and the text of the present document, the definition or use of the term in the present document shall prevail.

### BACKGROUND OF THE INVENTION

### Field of Invention

The present invention relates to an electrical removal device and a control method for the electrical removal device, and more particularly to an electrical removal device and a control method thereof for conveniently and safely removing hair with applied heat.

### Related Art

In our life, women generally need to remove unwanted hair from the body for beauty and social etiquette. Generally, mechanized cutting means for cutting hair are used, for example a shave razor. In use, the shave razor is uncomfortable to use, takes pain, and can't get a close shave, and results in different stubbles remaining thereon. Especially, the shave razor is easy to damage skin, which not only doesn't realize the beauty, but also leaves disfigurement security risk.

Recently, for resolving the above described questions, alternative hair removal devices that use an electrical or heat are provided, for example electrolysis or a heated wire. The electrolysis device has a low efficiency and high security risk. Therefore, the use of the heated wire or other similar structures to shave hair from a skin surface has been proposed.

However, a conventional heating wire for shaving hair utilizes high temperature heat, which usually damages the skin. Alternatively, since the heating wire is offset from the skin to prevent skin damage, unwanted stubbles are left. Moreover, in the fast-paced modern society, no user needs an inconvenient device. Therefor, a hair removal device having a super automation and a convenient operation is needed.

In addition, there's still other questions, such as some hot-curled stubble remaining on the skin after an heat removal process. The user needs to utilize a friction plate to wipe off the remaining stubble on the skin. It is so inconvenient.

At the same time, the heating wire can't be designed to be very closer to the skin, for preventing scalding the skin. So, there's some very shorter hair can't be removed , which looks so ugly, and touches so ruffly.

Therefore, a new electrical hair removal device and a control method for an electrical hair removal device are needed, which should be safely, conveniently, and effectively for hair removing.

### SUMMARY OF THE INVETION

Taking above into consideration, an object of an embodiment of the invention is to provide an control method for an electrical hair removal device, the electrical hair removal device including a heat generator having a heating wire formed thereon, including: step S11: starting the electrical hair removal device and let it go into a standby state; step S12: placing the heat generator on an hair removal needed area; and step S13: electrifying the heating wire to a predetermined temperature, for realizing hair removal with a constant heating temperature.

Another object of an embodiment of the invention is to provide that the step S13 further includes providing a switch button, in use, pressing and holding the switch button, the heating wire continuously emitting heat, and releasing the switch button, the heating wire stopping emit heat.

Another object of an embodiment of the invention is to provide that the heating wire is supplied DC power, and provides a constant heating power.

Another object of an embodiment of the invention is to provide that the heating wire can work can work at 200° C -400 ° C heating temperature, or the heating wire can work at three different adjustable working states, the first working state being 200° C -300 ° C heating temperature; the second working state being 300° C -350 ° C heating temperature; and the third working state being 350° C -400 ° C heating temperature.

Another object of an embodiment of the invention is to provide that a gap between the heating wire and a skin surface is greater than 0 mm and less than 3mm, in use.

Another object of an embodiment of the invention is to provide that step S13 further includes providing a sensor, when the sensor is triggered, the heating wire is electrified for heating.

Another object of an embodiment of the invention is to provide that the sensor is triggered to electrify the heating wire, when the heat generator retracts backwardly by contacting with a skin surface, and the sensor stops work for disconnecting the heating wire, when the heat generator resets by an elastic deformation of a spring.

Another object of an embodiment of the invention is to provide that step S13 further includes a timer or a calculagraph for automatically deactivating the heating wire after a predetermined time.

Another object of an embodiment of the invention is to provide that step S12 further includes a cutting element, which cooperates with the heat generator for removing the unwanted hair, the heat generator being used to burn out the hair and utilizes the heat to removing the hair root, the cutting element being used to cut hair. Another object of an embodiment of the invention is to provide that the heating generator is used to remove the longer hair, and the cutting element is used to remove the shorter hair.

Another object of an embodiment of the invention is to provide that step S13 further includes a switch key and a mode key, the switch key being used to switch the temperature scope of the heating wire, and the mode key being used to choose one of three different work modes, including the heat generator single working mode, the cutting element single working mode, and the heat generator and the cutting element cooperating mode.

Another object of an embodiment of the invention is to provide An electrical hair removal device, which includes a main body. The main body including a heat generator having a heating wire thereon, a supporting element and a printed circuit board (PCB); and a cover for accommodating the main body; wherein the heating wire is electrically connected to the PCB, which is electrified and heated to a predetermined temperature, for realizing hair removal with a constant heating temperature.

Another object of an embodiment of the invention is to provide that the PCB includes a conductive terminal, and the supporting element includes an electrical shock head, the conductive terminal and the electrical shock head being electrically connected when the heat generator are positioned on the skin.

Another object of an embodiment of the invention is to provide an electrical hair removal device that further includes a spring provided between the supporting element and the PCB, when the heating generator leaves away the skin surface, the electric shock head of the supporting element disconnects with the conductive terminal of the PCB by the elastic deformation of the spring.

Another object of an embodiment of the invention is to provide that the heating wire can provide a heating temperature from 250 ° C to 450° C, or provide three different working states, that the first working state is 250-300 ° C heating temperature; the second working state is 300-400 ° C heating temperature; and the third working state is 400-450 ° C heating temperature.

Another object of an embodiment of the invention is to provide that the main body further includes a cutting element, which cooperates with the heat generator for removing the unwanted hair, the heat generator is used firstly, and the cutting element is used sequentially.

Another object of an embodiment of the invention is to provide that the cutting element includes a cutting head and a motor , the motor driving the cutting head to move back and forward.

Another object of an embodiment of the invention is to provide that an electrical hair removal device further includes a comb, which includes a plurality of comb teeth, being used to comb the hair on the skin surface, for subsequently easy removal.

Another object of an embodiment of the invention is to provide that the backwardly retraction of the heat generator can bring a photoelectric trigger by a photoelectric sensor, or bring an electrical connection for is supplying a DC power to the heating wire.

Compared with the prior art, an embodiment of the electrical hair removal device allows an instant start upon use mode, i.e. only when the epilation starts, the electric heating wire automatically starts for hair removal, and as long as no hair removal needed or the electrical hair removal device leaves the skin, the heating wire can stop work. Therefore, its use is very safe and convenient. The embodiment of the invention provides two ways to realize the instant start upon use mode. One is by the switch button. The user only need press the switch button when depilation is needed. The heating wire is electrified for removing the hair through heat. When the hair removal is finished, the user only need release the switch button, and the electrical hair removal device returns to the standby state. This way utilizes a coordination and collaboration of human's hand and body to realize a human oriented use design. The other way uses a photoelectric sensor to control the heating wire for removing hair. As long as the contact terminal contacts with the skin surface, the photoelectric sensor is instantly triggered and let the heating wire to be electrified for heating. This way utilizes an auto-sensing method to achieve the instant start upon use mode, which makes the use of the electrical hair removal device more accurate and secure. In addition, the embodiment of the invention provides multiple grades heating temperature ranges, the user can choose the right heating temperature range according to their own needs. For coarse hair, the user can choose a higher heating temperature grade. For fine hair, the user can choose a lower heating temperature grade. For users, this multiple heating selection settings take more practical and convenient operation. And the heating temperature is thermostat, which is achieved by providing the DC constant voltage power supply to the heating wire. So, the control of the electrical hair removal device becomes simple. The depilatory area is heated evenly, and the depilatory efficiency is better. An embodiment of the electrical hair removal device utilizes a removable heat generator. When the heat generator is needed to repair or replace, the user only need to pull it down. This operation is easy to use and maintain. Furthermore, in use state, the heating wire is not directly contact with the skin, so that the electrical hair removal device can prevent the high temperature of the heating wire burning the skin. In addition, the electrical hair removal device utilizes the heating wire blowing the hair and utilizes a high temperature to crimp hair roots thereof that not being in contact with the electric wire. Thus, a regeneration rate of the hair can be deferred.

In addition, the present invention electrical hair removal device further provides a new solution, which utilizes the heat generator and the cutting element cooperation for removing the hair. The heat generator is used to remove the longer hair rapidly and painlessly, and the cutting element is used to cut the shorter hair or the remaining hot-curled hair immediately, after heat-removal process executed by the heat generator. Because the hot-curled hair is very soft, the cutting element can mechanically cut the hot-curled hair rapidly and painlessly. The cooperation of the heating element 306 and the cutting element can completely remove the hair on the skin , no matter the longer hair or the shorter hair.

At the same time, all kinds of work modes are provided for different user's needs. In the first work mode, the heat generator works first, and then the cutting element works sequentially. This work mode can utilize the heat generator to remove the longer hair firstly, and then utilize the cutting element for removing the remaining hair on the skin , which can realize a completely hair removal. In the second work mode, only the heat generator is drove. This work mode is suitable for low requirements. In the third work mode, only the cutting element is drove. Sometimes, the hair on the skin is very short, which looks unclean and unsmooth. But the heat generator can't be effective to the short hair. So, the cutting element can finish the work perfectly.

Other novel features and advantages will become apparent from the following detailed description of preferred and exemplary embodiments when taken in conjunction with the accompanying drawings.

### BRIEF DESCRIPTION OF THE DRAWINGS

FIG.1 is an isometric diagram of an electrical hair removal device according to a first exemplary embodiment of the invention.
FIG. 2 is an isometric exploded diagram of the electrical hair removal device of FIG.1.

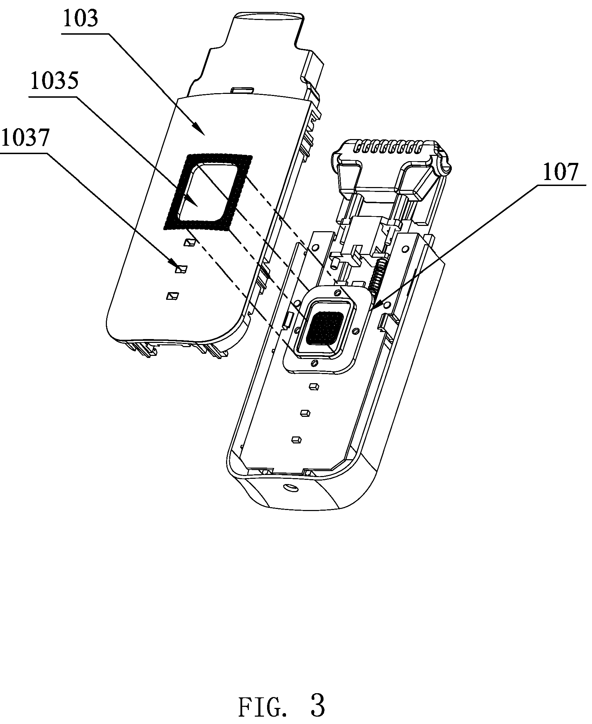

FIG.3 depicts an isometric view of the top cover coupling to the main body.
FIG.4 is an isometric exploded diagram of the main body of FIG.2.
FIG.5 depicts an assembling isometric view of the heat generator, the supporting element and the fixing seat of FIG.4.
FIG.6 depicts an isometric view of the heat generator of FIG.4.
FIG.7 depicts an isometric view of the supporting element of FIG.4.
FIG.8 depicts an assembling isometric view of the heat generator and the supporting element of FIG.4.
FIG.9 depicts an isometric view of the fixing seat of FIG.4.
FIG.10 depicts an assembling isometric view of the supporting element and the fixing seat of FIG.4.
FIG.11 depicts an isometric view of the PCB of FIG.4.
FIG.12 depicts a schematic circuit diagram of a control system integrated on the PCB of FIG.4.
FIG.13 depicts a schematic circuit diagram of the integrated amplifier circuit of FIG.12.
FIG. 14 depicts a schematic circuit diagram of the switch circuit of FIG. 12.
FIG. 15 depicts a schematic circuit diagram of the driving circuit of FIG. 12.
FIG.16 is a flowchart of a control method for an electrical hair removal device according to a second exemplary embodiment of the invention.
FIG. 17 is a flowchart of a control method for an electrical hair removal device according to a third exemplary embodiment of the invention.
FIG.18 is an isometric exploded diagram of an electrical hair removal device according to a fourth exemplary embodiment of the invention.
FIG. 19 is an isometric exploded diagram of the main body of FIG.18.
FIG.20 depicts an isometric view of the comb of FIG.19.
FIG.21 depicts an isometric view of the ferrule of FIG.19.
FIG.22 depicts an isometric view of the heat generator of FIG.19.
FIG.23 depicts an isometric view of the cutting element of FIG.19.
FIG.24 depicts an isometric exploded diagram of the cutting element of FIG.19.
FIG.25 depicts an isometric view of the motor of FIG.24.
FIG.26 depicts an isometric view of the PCB of FIG.19.
FIG.27 shows a flowchart for a use method for the electrical hair removal device of FIG.18.

### DETAILED DESCRIPTION OF THE INVENTION

Reference will now be made to the drawings to describe preferred and exemplary embodiments in details.

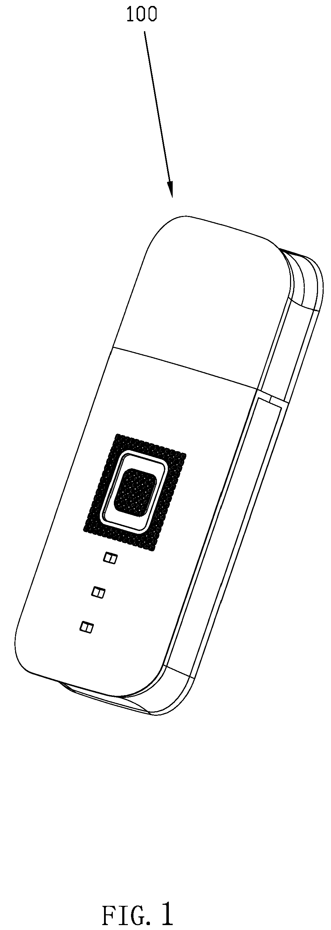

Referring to FIG.1, an electrical hair removal device according to a first embodiment of the invention is shown. The electrical hair removal device 100 is shaped like a rectangular, having four arc-shaped transition designs at four corners, respectively. The electrical hair removal device 100 has a length greater than its width and thickness, the length being 2-5 times the width, and being 3-10 times the thickness, which assures an easier holding for a user. For maintaining an ergonomic design, the length of the electrical hair removal device 100 is almost similar to that of a human hand, the width is less than that of any part of a human body, and the thickness is designed to be as thinner as possible. Thus, the electrical hair removal device 100 has a compact structure, facilitates the use, and assures a human oriented design.

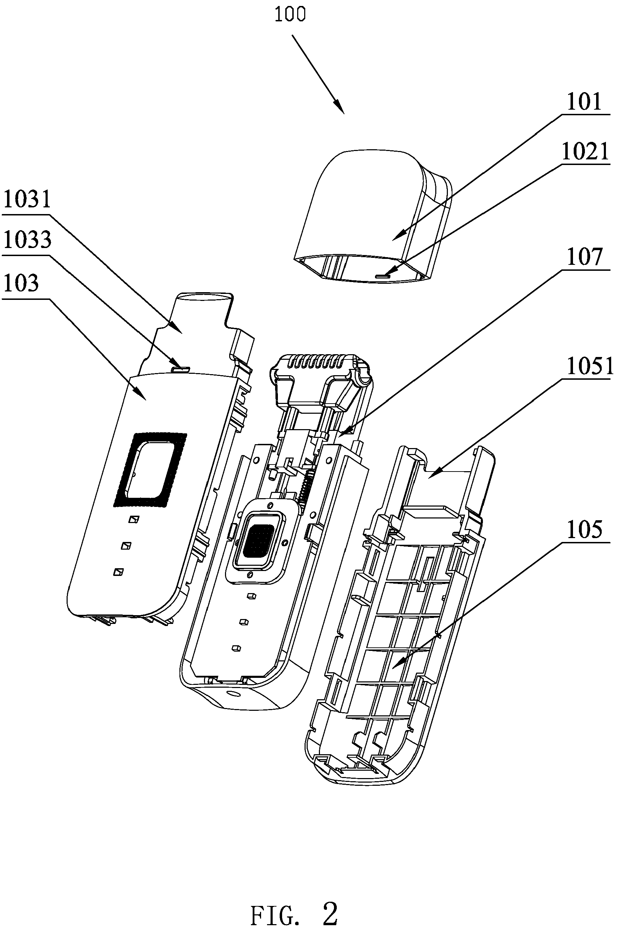

Referring to FIG. 2, the electrical hair removal device 100 includes a main body 107, a top cover 103, a bottom cover 105, and a cap 101. The main body 107 is used to assemble all elements of the electrical hair removal device 100. The top cover 103 and the bottom cover 105 are respectively disposed at a top side and a bottom side of the main body 107, for ornamenting and covering the main body 107. In assembly, the top cover 103 and the bottom cover 105 cooperate to accommodate the main body 107 and other elements of the electrical hair removal device 100 thereon. The cap 101 is used to cover and accommodate a head part (not labeled) of the main body 107, a head part (not labeled) of the top cover 103, and a head part (not labeled) of the bottom cover 105. Therefore, the cap 101, the top cover 103, and the bottom cover 105 are assembled together to accommodate the main body 107, and form a rectangular structure.

The top cover 103 has a top baffle 1031. The bottom cover 105 has a bottom baffle 1051. The top baffle 1031 and the bottom baffle 1051 are correspondingly opposite to each other, which are used to cooperate with the cap 101. In addition, the top baffle 1031 and the bottom baffle 1051 are used to protect a heating part of the main body 107 for assuring a safety use.

The cap 101 has a couple of clips 1021, on two inner sides (not labeled) thereof, which cooperate with two grooves 1033, respectively disposed on the top baffle 1031 and the bottom baffle 1051. In assemble, the cap 101 can be installed on the top cover 103, the bottom cover 105, and the main body 107 through block-joints of the two clips 1021 and the two grooves 1033, respectively.

The top cover 103 further includes a button hole 1035, formed at a middle region, a plurality of lamp holes 1037, sequentially formed on an under-part thereof. In addition, an ornamental figure can be provided on the top cover 103, even on the bottom cover 105 and the cap 101 according to the user's fancy.

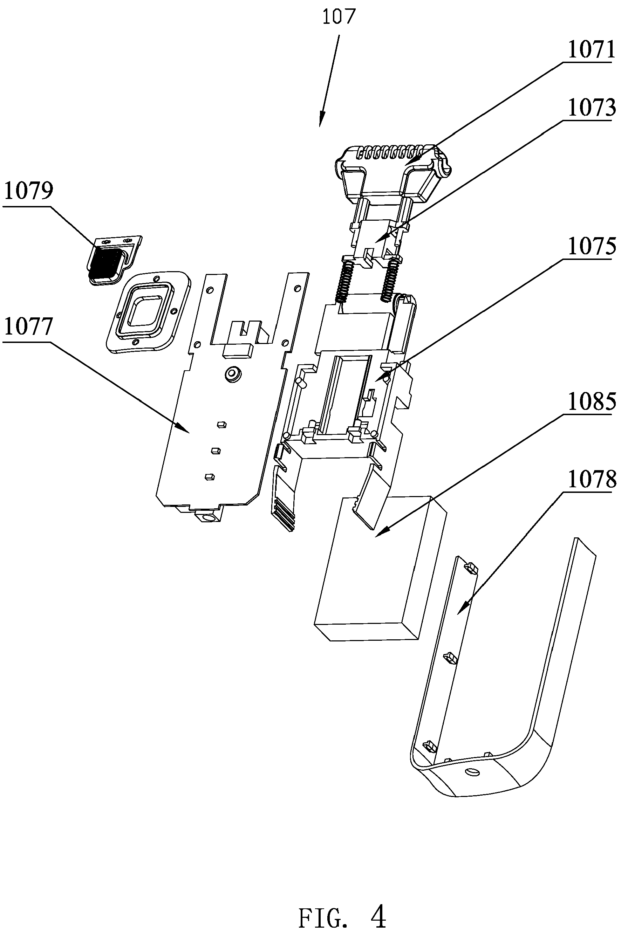

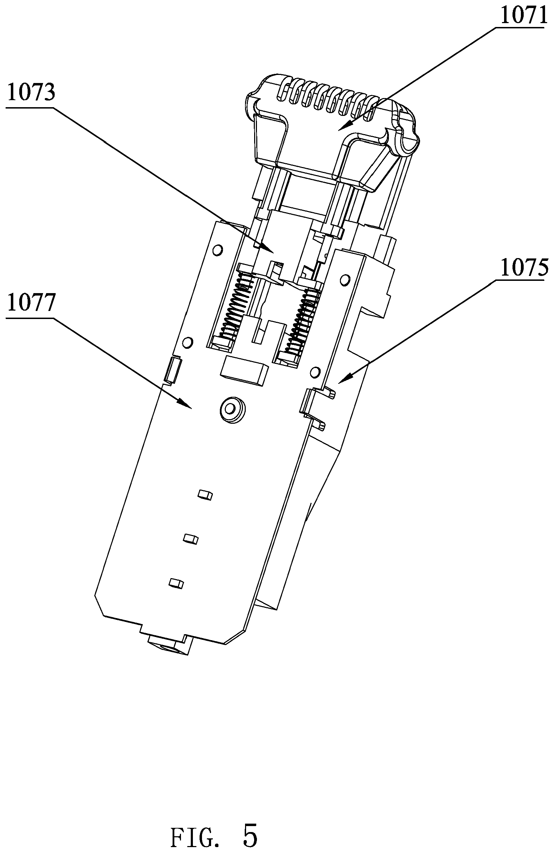

Referring to FIG. 4 and FIG.5, the main body 107 has a heat generator 1071, a supporting element 1073, a fixing seat 1075, and a printed circuit board (PCB) 1077. The heat generator 1071, the supporting element 1073, and the fixing seat 1075 are sequentially assembled from top to bottom. The PCB 1077 is fixed on the fixing seat 1075. The heat generator 1071 is connected to supporting element 1073 fixed on the fixing seat 1075. An ornamental frame 1078 is further provided between the three peripheral sides of the fixing seat 1075, which cooperates with the top cover 103 and the bottom cover 105 to realize wrapping the fixing seat 1075. The main body 107 further includes a button panel 1079, corresponding to the button hole 1035 on the top cover 103, which is functioned as a control panel. The button panel 1079 is designed with a non-planar rough surface for antiskid. The fixing seat 1075 further accommodates a battery for supplying power to the electrical hair removal device 100, which electrically connects with the heat generator 1071.

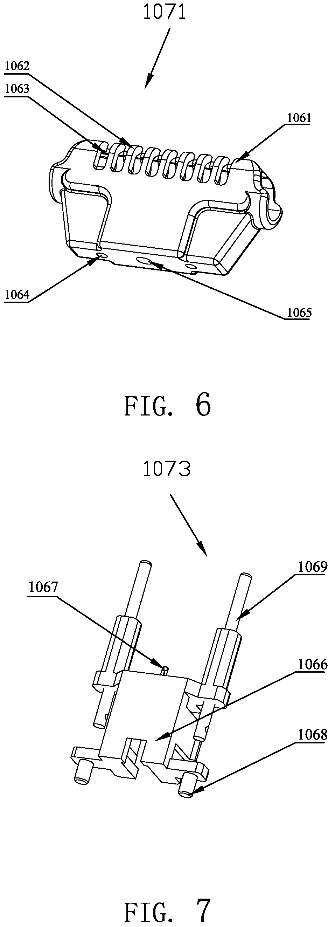

Referring to FIG.6, the heat generator 1071 includes a contact terminal 1061 and a heating wire 1063. The contact terminal 1061 is used to directly contact with the skin for flattening a surface of the skin. The contact terminal 1061 includes two pieces symmetrical contact pressing teeth 1062, defining a gap therebetween. The heating wire 1063 is accommodated in the gap. The position of the heating wire 1063 is lower than that of the contact pressing teeth 1062. That is, when the contact pressing teeth 1062 contacts with the skin at an initial stage, the heating wire 1063 doesn't contact with the skin. In use stage, after the two contact pressing teethes 1062 press the skin downwardly and flatten the hair thereon, the heating wire 1063 begins cutting the hair. So that, the electrical hair removal device 100 can prevent scalding the skin, which utilizes the heating wire 1063 to burn out the hair and utilizes the heat to remove the hair root, and prohibits a hair re-growth speed. The heat generator 1071 has two conductive holes 1064 at one end, which transition fits at a top end of the supporting element 1073 through the two conductive holes 1064. Thus, the heat generator 1071 can be pulled down. Of course, as long as the heat generator 1071 can be easily pulled down, other fixing types also are accepted, such as magnetic type, snap type, etc. The two conducting holes 1064 respectively accommodate two metal pieces (not shown), which connects to two terminals of the heating wire 1063, i.e. positive and negative polarities of the heating wire 1063. When the heat generator 1071 is fixed at the supporting element 1073, the metal pieces of the conductive holes 1064 are electrically connected to a conductive element (not shown) of the supporting element 1073. The heat generator 1071 further includes a lamp hole 1065 for receiving an illumination light, between the two conductive holes 1064.

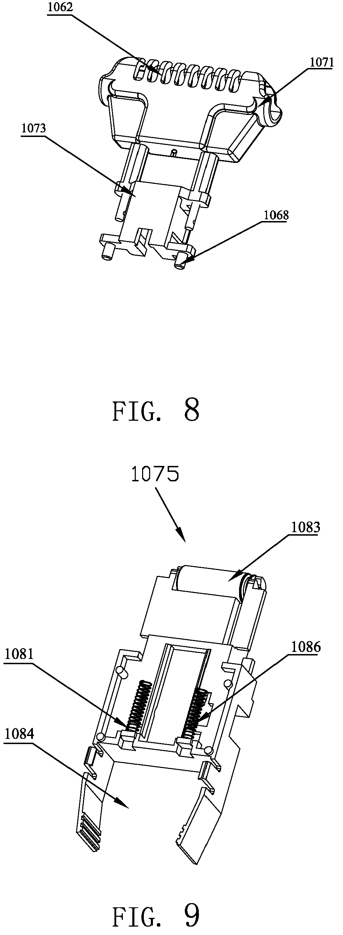

Referring to FIG.7 and FIG.8, the supporting element 1073 includes a supporting body 1066, two electrical shock heads 1068, two conductive posts 1069, and an illumination light 1067. The two conductive posts 1069 are positioned at a top end of the supporting body 1066, and the two electrical shock heads 1068 are positioned at a bottom end of the supporting body 1066, and the illumination light 1067 is disposed between the two conductive posts 1069. The two electrical shock heads 1068 elastically touch the fixing seat 1075 for realize electrical conduct. The illumination light 1067 corresponds to the lamp hole 1065 of the heat generator 1071, which is a blue LED (light emitting diode) in this embodiment for efficiently removing hair and illuminating a hair removal state. In addition, the illumination light 1067 can be other type light source, and can also be disposed at other position, so long as it can illuminate the hair removal region.

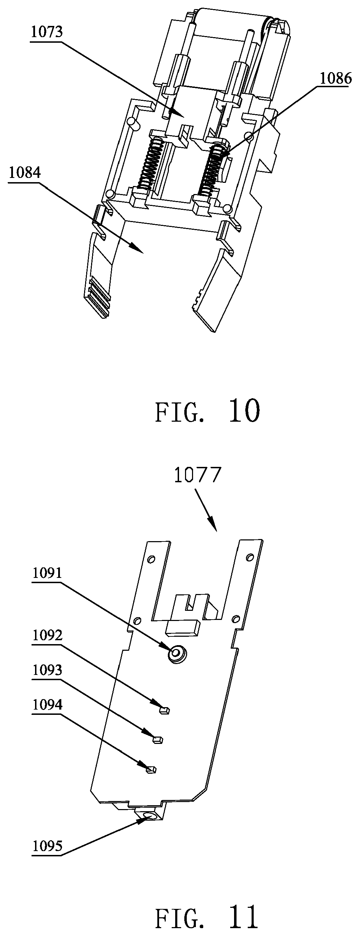

Referring to FIG. 9 and FIG.10, the fixing seat 1075 is used to fix the supporting element 1073 and the heat generator 1071 at its front end, which includes a battery cavity 1084 at its rear end, two conductive terminals 1081, two springs 1086, a guiding wheel 1083 at its front end. The battery cavity 1084 accommodates the battery 1085 for supplying power to the heat generator 1071. The guide wheel 1083 corresponds to the contact terminal 1061, as a movement fulcrum of the contact terminal 1061. The guide wheel 1083 can help the contact terminal 1061 to smoothly move and flatten the hair removal region. The two conductive terminals 1081 connect to an anode and a cathode of the battery 1085, respectively, positioned at a top side of the battery cavity 1084. The two springs are respectively provided between the two conductive terminals 1081 and the two electrical shock heads 1068. At the initial state, the conductive terminal 1081 doesn't touch the electrical shock heads 1068, and at the use state, the conductive terminal 1081 touches and electrically connects with the electrical shock heads 1068 after an external force are provided to the springs 1086 to produce an elastic deformation. Thus, the power of the battery 1085 is provided to the head generator 1071 through the conductive terminals 1081, the electrical shock heads 1068 and the conductive posts 1069. When the external force is reduced or doesn't exist, the springs 1085 rebounds, and the electrical shock heads 1068 separates from the conductive terminals 1081. Thus, the heat generator 1071 disconnects with the battery 1085.

Referring to FIG. 11, the PCB 1077 covers the fixing seat 1075, and electrically connects the battery 1085 of the battery cavity 1084, for controlling the output voltage of the battery 1085. The PCB 1077 includes a switch button 1091, a blue start indicating light 1092, a red use indicating light 1093, and a green charge indicating light 1094. The switch button 1091 corresponds to the button hole 1035 of the top cover 103. The blue start indicating light 1092, the red use indicating light 1093, and the green charge indicating light 1094 respectively correspond to the plurality of lamp holes 1037. The PCB 1077 further includes a charging socket 1095 for charging the battery 1085.

In assembly, firstly, the supporting element 1073 is assembled to the fixing seat 1075, with the two electrical shock heads 1068 plugging into the two springs 1086 of the fixing seat 1075, respectively. So that, the supporting element 1073 is limited to slid along a vertical direction, and the conductive posts 1069 are mounted on the fixing seat 1075 for electrical connection between the heat generator 1071 and the conductive posts 1069. Secondly, the PCB 1077 is mounted on the fixing seat 1075 though screw fixing mode or snap fixing mode. Thirdly, the button panel 1079 is formed on the PCB 1077 for covering the switch button 1091. After these processes, the assembly of the main body 107 are finished.

Consecutively, the ornamental frame 1078 is assembled to three peripheral sides of the main body 107. The top cover 103 is fixed at a top side of the main body 107, and the bottom cover 105 is fixed at a bottom side of the main body 107. Thus, the top cover 103, the bottom cover 105, and the ornamental frame 1078 form an accommodating cavity for accommodating the main body 107 therein.

Finally, the heat generator 1071 is plugged on the supporting element 1073. Because the heat generator 1071 transition fits at a top end of the supporting element 1073, the disassembly and assembly department is easier. So, for maintaining different needs of different user, the heat generator 1071 can be designed to different sizes. After that, the cap 101 can be provided to accommodate the heat generator 1071 therein through the cooperation of the block-joints of the two clips 1021 and the two grooves 1033.

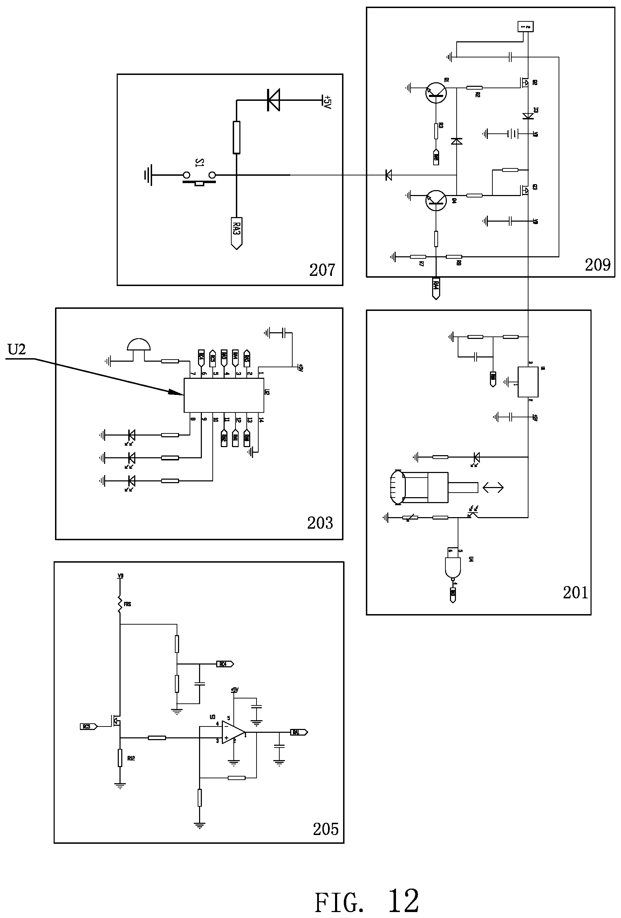

FIG. 12 depicts a schematic circuit diagram of a control system 200 integrated on the PCB 1077 of FIG.4, the control system 200 includes a voltage regulator circuit 201, a control circuit 203, an integrated amplifier circuit 205, a switch circuit 207, and a driving circuit 209. The voltage regulator circuit 201, the integrated amplifier circuit 205, the switch circuit 207, and the driving circuit 209 are electrically connected to the control circuit 203. The voltage regulator circuit 201 outputs a voltage to the control circuit 203. The control circuit 203 provides analog signals to the integrated amplifier circuit 205 and the driving circuit 209 after the switch circuit 207 turns on, for controlling the heat generator 1071.

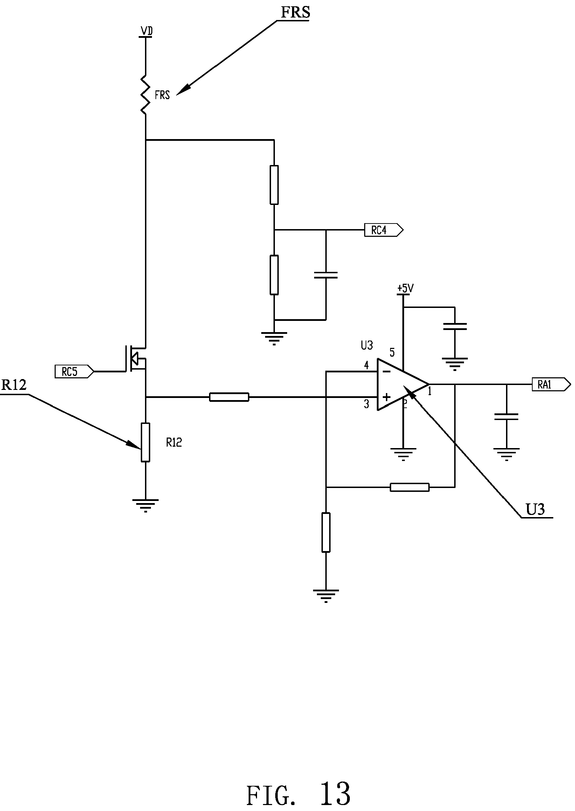

FIG.13 depicts a schematic circuit diagram of the integrated amplifier circuit 205 of FIG. 12. The integrated amplifier circuit 205 drives the heating wire 1063. Through a synchronous ratio amplifier circuit U3, a voltage signal of a resistance R12 is inputted to the microcontroller U2, for detecting the length of the heating wire 1063 and providing different voltages to different length heating wires 1063. The heating wire 1063 can have two different lengths, longer or shorter for removing different length hairs. The longer heating wire 1063 is used to remove longer hair, and the shorter heating wire 1063 is used to remove shorter hair. The integrated amplifier circuit 205 provides two different voltages to the heating wire 1063 according to the detecting result. Thus, when the guiding wheel 1083 moves along the skin, the heat generator 1071 can produce different heat temperatures for different length hair.

As above described, for different lengths heating wire 1063, the control circuit 203 provides different drive voltages, so that the different lengths heating wires 1063 have different hair removal temperatures at different usage state. When the length of the heating wire 1063 is longer, the electrical hair removal device 100 provides a heating temperature of 250° C -450 ° C, and can work about 45 minutes. When the length of the heating wire 1063 is shorter, the electrical hair removal device 100 provides a heating temperature of 200° C -400 ° C, and can work time of 85 minutes.

In addition, for an alternative, the voltage regulator circuit 201 can provide different voltages to the heating wire 1063. Thus, the longer heating wire 163 has a heating temperature scope of 250° C -450 ° C, and the shorter heating wire 163 has a heating temperature scope of 200° C -400 ° C. Accordingly, the PCB 1077 can design multi-position selective buttons (not shown), every position selective button can provide a predetermined driving voltage. Because the control system 200 adopts DC power supply, the power supply current is constant. When the driving voltage is changed, the heating power is changed, and ultimately the different heating temperatures are provided. For example, the PCB 1077 sets up three-position selective buttons thereon. The longer heating wire 1063 can work at three working states. At the first state, the heating wire 1063 is provided to 4.6V supply voltage, and 250° C -300 ° C heating temperature. At the second state, the heating wire 1063 is provided to 5.2V supply voltage, and 300° C -400 ° C heating temperature. At the third state, the heating wire 1063 is provided to is 5.9V supply voltage, and 400° C - 450 ° C heating temperature. The shorter heating wire 1063 can work at three working states, too. At the first state, the heating wire 1063 is provided to 2.7V supply voltage, and 200° C -300 ° C heating temperature. At the second state, the heating wire 1063 is provided to is 3.0V supply voltage, and 300° C -350 ° C heating temperature. At the third state, the heating wire 1063 is provided to is 3.2V supply voltage, 350° C -400 ° C heating temperature. For different regions of the human body or different users, different working states can be chose. When we need to remove men's coarser hair, we can select the 3^{rd} working state at beginning. When we need to remove women's fine hair, we can select the 1^{rd} working state at beginning. So many selections design allows consistent with all kinds of needs of the user.

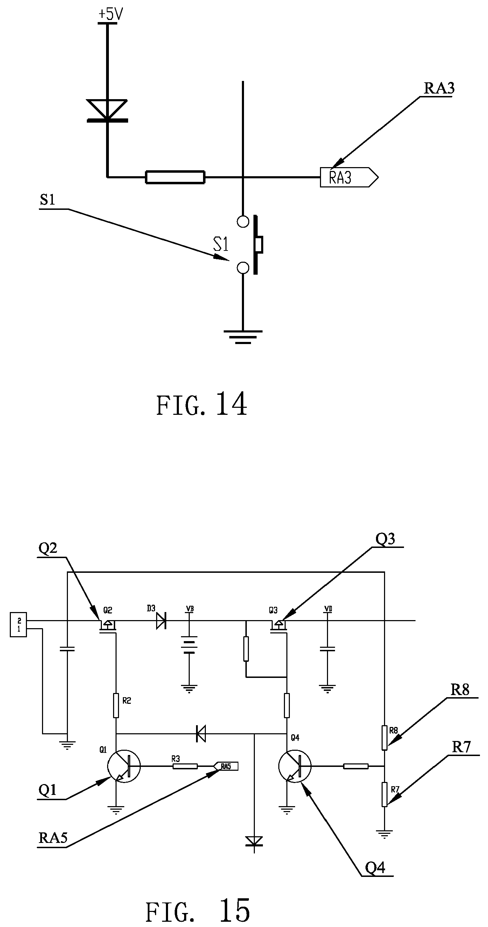

FIG.14 depicts a schematic circuit diagram of the switch circuit 207 of FIG. 12. When the user presses the switch button 1091, a corresponding switch S1 of the switch circuit 207 turns on. A MOSFET Q3 of the driving circuit 209 turns on. When a power supply of the microcontroller U2 is 5V, a pin RA3 of the switch circuit 207 converts from high level to low level, and the electrical hair removal 100 starts to work. In use state, when the user presses the switch button 1091 again, the microcontroller U2 detects a level change of the pin RA3. Thus, the electrical hair removal 100 turns off.

FIG.15 depicts a schematic circuit diagram of the driving circuit 209 of FIG. 12. The driving circuit 209 drives the control circuit 203. When the driving circuit 209 doesn't connect to a charger (not shown) and the switch S1 is pressed, MOSFET Q3 turns on and the microcontroller U2 let a pin RA5 to convert to high level. Thus, a transistor Q1 turns on and electrically connects the MOSFET Q2 and the MOSFET Q3 for locking power. When a charger (not shown) is provided, a charging voltage through partial pressure by two resistors R8, R7 is used to drive a transistor Q4, the MOSFET Q3 is turned on. When the microcontroller U2 is +5 V, the microcontroller U2 makes pin RA5 being high level, and the transistor Q1 and the MOSFET Q2 turns on. The battery 1085 is charged.

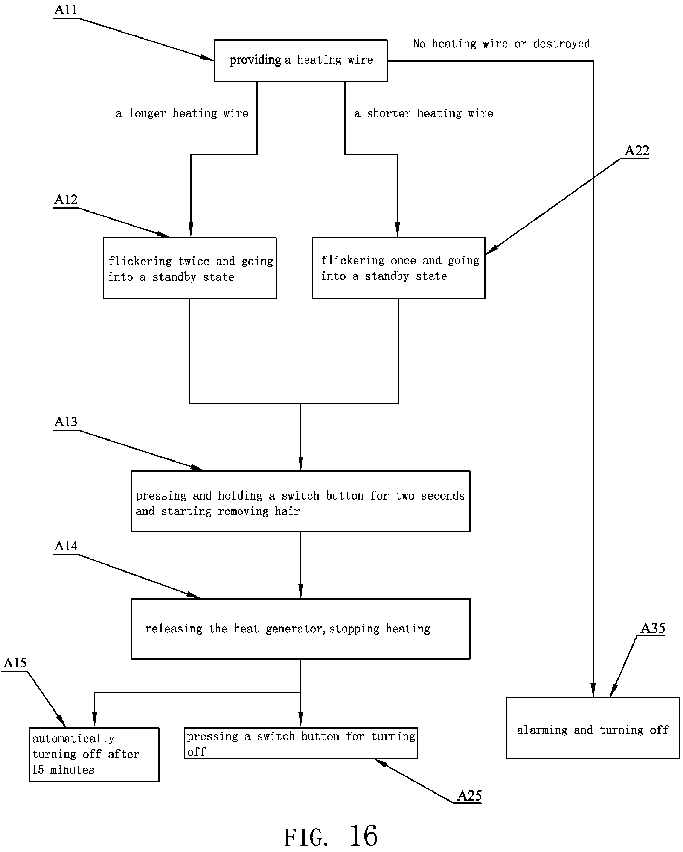

FIG.16 is a flowchart of a control method for the electrical hair removal device 100 according to a second exemplary embodiment of the invention.

In the 1^{st} step, the user put the heat generator 1071 on the conductive posts 1069 of the supporting element 1073. The user can choose different heating wires 1063, such as the longer heating wire, or the shorter heating wire. The heating temperature for the longer heating wire 1063 is 250° C -450° C. The heating temperature for the shorter heating wire 1063 is 200° C -400° C. When the hair is long and coarse, the user should choose the longer heating wire 1063. When the hair is short and fine, the user should choose the shorter heating wire 1063. In addition, the heating wire 1063 has a variety of types, is not limited to the above-described two types. The user can choose an appropriate heating wire, working at 200° C -450 ° C temperature range.

In the 2^{nd} step, after the user presses the switch button 1091 two seconds, the electrical hair removal device 100 turns on. The integrated amplifier circuit 205 begins to check the length of the heating wire 1063, and judges it for providing different driving voltage. For easily distinguishing different states, the electrical hair removal device 100 designs the blue start indicating light 1092, the red use indicating light 1093, and the green charge indicating light 1094. When the integrated amplifier circuit 205 judges that the heating wire 1063 is longer, the blue start indicating light 1092 flickers twice, and a drop sound rings out. The electrical hair removal device 100 is into a standby state. When the integrated amplifier circuit 205 detects that the heating wire 1063 is shorter, the blue start indicating light 1092 flickers once, and a drop sound rings out. The electrical hair removal device 100 is into a standby state. When the integrated amplifier circuit 205 doesn't detect the heating wire 1063, the electrical hair removal device 100 judges that the heating generator 1071 is not provided, or the heating wire 1063 is destroyed. Therefore, the red use indicating light 1093 flickers twice, and an alarm sound rings out. Thus, the user should turns off the power.

In the 3^{rd} step, if the heating wire 1063 is mounted right, the control system 200 goes into the usage state. The user presses the switch button 1091 long than two seconds, the switch circuit 207 converts the electrical hair removal device 100 to the use state. Simultaneously, the red use indicating light 1093 illuminates, and the heating wire 1063 is heated and is aglow, which reminds that the heating wire 1063 has reached a predetermined temperature. In use stage, the user should press and hold the switch button 1091, and place the heat generator 1071 to a hair removal needed area. Conversely, once the switch button 1091 is released, the switch circuit 207 turns off the heating wire 1063. The heating wire 1063 stops heating. The red use indicating light 1093 turns off. The electrical hair removal device 100 returns to the standby state. At this point, the user is reminded that the heating wire 1063 has no heat, can be taken away form the hair removal needed area. The same operation can be done, if the user needs to removal hair at other areas. The user should presses and holds the switch button 1091 again to start the heating wire 1063, until the hair has removed. After the hair removal is completed, the user should release the switch button 1091, take out the electrical hair removal device 100 away, and let the electrical hair removal device 100 return to standby state. Repeating the above-described process, the user can control to remove hairs at various areas of the skin, according to the needs. Because the electrical hair removal device 100 utilizes a single button operating mode, the operation is very convenient and freedom.

In the 4^{th} step, after the unwanted hair is completely removed, the user just need to tap the switch button 1091, for disconnecting the heating wire 1063 and the PCB 1077 by the switching circuit 207. Then, the heating wire 1063 stops heating, and the blue start indicating light 1092 turns off. The user is reminded that the electrical hair removal device 100 is into an off-state. In addition, in order to reflect the security and user-friendly design, the switch circuit 207 is set on a timer for deactivating the electrical hair removal device 100 at a set time. The electrical hair removal device 100 self-shutdowns after it works 15 minutes. This design can prevent the work time is too long resulting a lower cooling efficiency, and a potential safety hazards.

Specially, in use, when the heat generator 1071 contacts with the skin, a depilation gap between the heating wire 1063 and the skin is about 0.1 mm, mainly to prevent the high temperature of the heating wire 1063 to burn the skin. In addition, the electrical hair removal device 100 utilizes the heating wire 1063 to burn out the hair and utilizes a high temperature to crimp hair roots thereof. Thus, the hair roots don't contact with the heating wire 1063. Thus, a regeneration rate of the hair can be deferred. The operation theory for the electrical hair removal 100 is different from the conventional shaver, which directly cuts off the unwanted hair. The depilation gap of 0.1 mm can assure a safe operation and delay the hair regeneration rate. Of course, the depilation gap can be larger or smaller, generally greater than 0 mm and less than 3 mm.

Furthermore, after a use period of the electrical hair removal device 100, the battery 1085 needs to be charged. After start charging, the drive circuit 209 regulators an input voltage to a rated voltage. The green charge indicating light 1094 continues to illuminate, reminding the user that the electrical hair removal device 100 is in the charging state. After a continuous charging period, the green charge indicating light 1094 turns off, reminding the user that the charging of the battery 1085 has been completed.

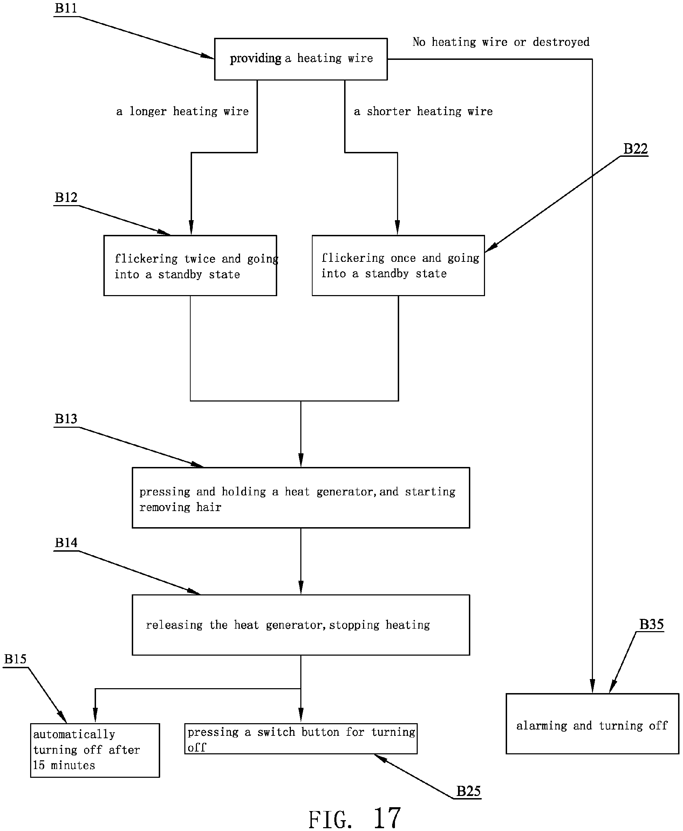

FIG. 17 is a flowchart of a control method 600 for the electrical hair removal device 100 according to a third exemplary embodiment of the invention. The control method 600 is similar to the control method 500 except that the third step.

In the third step of the control method 600, when the control system 200 is in the standby state, the heat generator 1071 is placed at a hair removal needed surface of the skin. When the heat generator 1071 retracts backwardly due to the contact terminal 1061 conflicting with the skin, the heat generator 1071 brings the whole supporting element 1073 retracts backwardly. After the heat generator 1071 retracts to a predetermined position, i.e. the top of the top heating generator 1071 falls on a same level of the guiding wheel 1083, the electric shock head 1068 of the supporting element 1073 contacts with the conductive terminal 1081 of the fixing seat 1075, for electrical conductivity. The battery 1085 can provide a stable power supply to the heating wire 1063. At the same time, the red use indicating light 1093 illuminates, and the heating wire 1063 glows and emits heat, which reminds the user that the heating wire 1063 has reached a predetermined temperature, and the heating wire 1063 can be used to remove hair. The user only needs to move the electrical hair removal device 100 back and forth and ensures that the electrical hair removal device 100 does not leave the skin surface. The electrical hair removal device 100 can continually remove the hair. Conversely, once the heating generator 1071 leaves away the skin surface, the electric shock head 1068 of the supporting element 1073 disconnects with the conductive terminal 1081 of the fixing seat 1075 by the elastic deformation of the springs 1086. At the same time, the heating wire 1063 stops emitting heat, the red use indicating light 1093 turns off, and the electrical hair removal device 100 goes back to the standby state. The same operation can be done, if the user needs to remove hair at other areas. The user only needs to place the heat generator 1071 on the hair removal needed region, and the heating wire 1063 can be electrified for removing the hair. When the user lets the heat generator 1071 far away from the skin surface, the electrical hair removal device 100 return back to the standby state. Repeating the above-described processes, the user can control to remove hairs at various areas of the skin, according to the needs. Because the electrical hair removal device 100 utilizes a touch trigger mode, the operation is very convenient and freedom. As an alternative embodiment, except the touch trigger mode for realizing an electrically conductivity of the heating wire 1063, a photoelectric sensor can be placed on the fixing seat 1075 or the PCB 1077. When the contact terminal 1061 brings the supporting element 1073 to retract backwardly, the photoelectric sensor is triggered, and let the control circuit 203 to provide the power supply, and begin the hair removal. When the hair removal is completed, the heat generator 1071 and heating wire 1073 resets on the elastic deformation of the springs 1086. The photoelectric sensor disconnects the power supply circuit of the heating wire1063. The heating wire 1063 stops emit heat. The electrical hair removal device 100 returns to the standby state. The alternative embodiment is more convenient and practical, and also has a relatively higher control accuracy.

As an alternative embodiment for better actuating the heating wire 1063, a gyroscope (not shown) can be provided on the heat generator 1071. When the heat generator 1071 is set on the skin of the user and the gyroscope detects an incline of the heat generator 1071, the heat generator 1071 is driven to heat up to the predetermined temperature for hair removal. When the electrical hair removal device 100 is positioned vertically, the heat generator 1071 stops heating. The motion detection mode for actuating the heating wire is convenient for the user's habit.

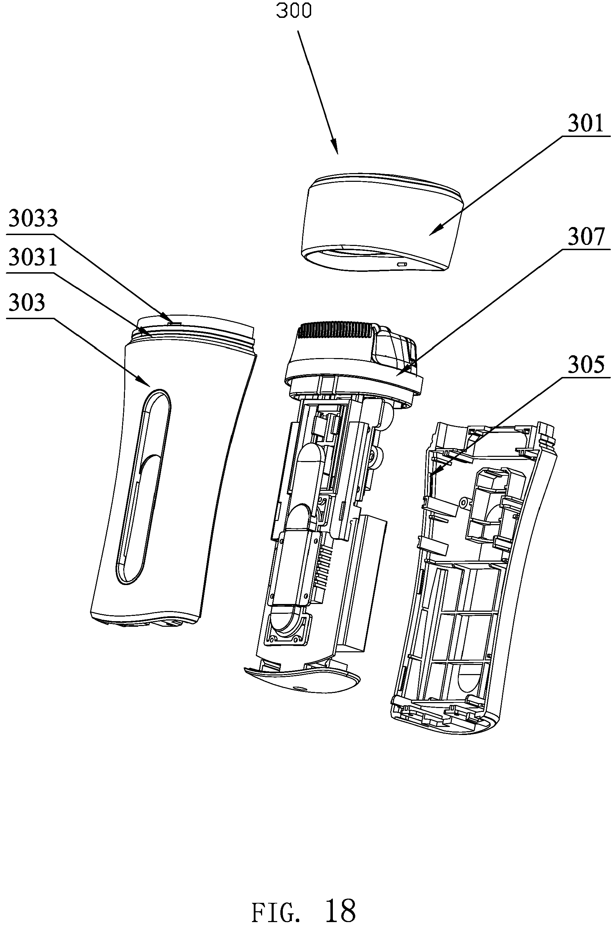

Referring to FIG.18, an isometric diagram of an electrical hair removal device 300 according to a fourth exemplary embodiment of the invention. The electrical hair removal device 300 includes a main body 307, a top cover 303, a bottom cover 305, and a cap 301. The main body 307 is used to assemble all elements of the electrical hair removal device 300. The top cover 303 and the bottom cover 305 are respectively disposed at a top side and a bottom side of the main body 307, for ornamenting and covering the main body 307. In assembly, the top cover 303 and the bottom cover 305 cooperate to accommodate the main body 307 and other elements of the electrical hair removal device 300 thereon. The cap 301 is used to cover and accommodate a head part of the main body 307, a head part of the top cover 303, and a head part of the bottom cover 305. Therefore, the cap 301, the top cover 303, and the bottom cover 305 are assembled together to accommodate the main body 307, and form a rectangular structure with a beautiful ornament.

The top cover 303 has a top baffle 3031. The bottom cover 305 has a bottom baffle (not shown). The top baffle 3031 and the bottom baffle are correspondingly opposite to each other, which are used to cooperate with the cap 301. The top baffle 3031 includes a groove 3033, and the bottom baffle includes a groove (not shown) too, which the two grooves are used to make the cap 301 firmly engaging at the top cover 303 and the bottom cover 305 and not easy to fall off.

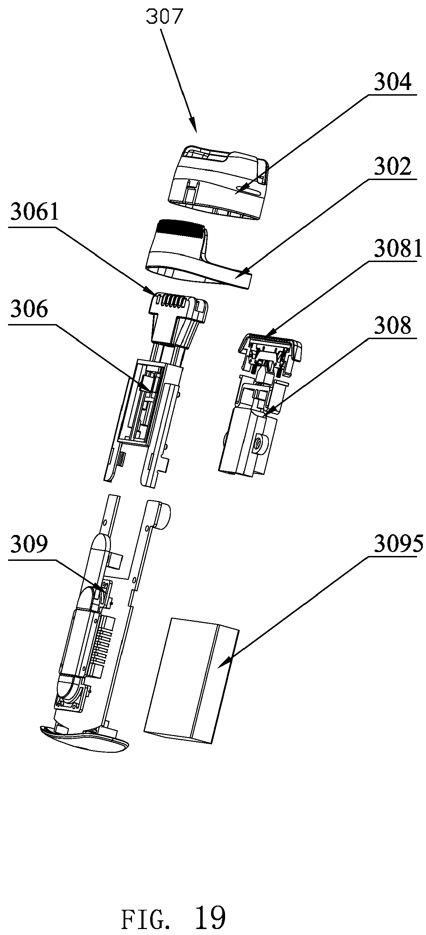

Referring to FIG. 19, the electrical hair removal device 300 further includes a head 304, a ring-shaped comb 302, a heating element 306, a cutting element 308, a PCB 309 and a power supply 3095. The main body 307 is strip-shaped. The head 304 with the ring-shaped comb 302 thereon is positioned at a front end of the main body 307, for accommodating a heat generator 3061 of the heating element 306 and a cutting head 3081 of the cutting element 308. The heating element 306 and the cutting element 308 electrically connect to the PCB 309, which provide a power from the power supply 3095. The heating element 306 is used to burn out the hair and utilizes the heat to remove the hair root, and prohibits a hair re-growth speed. The cutting element 308 is used to cut hair.

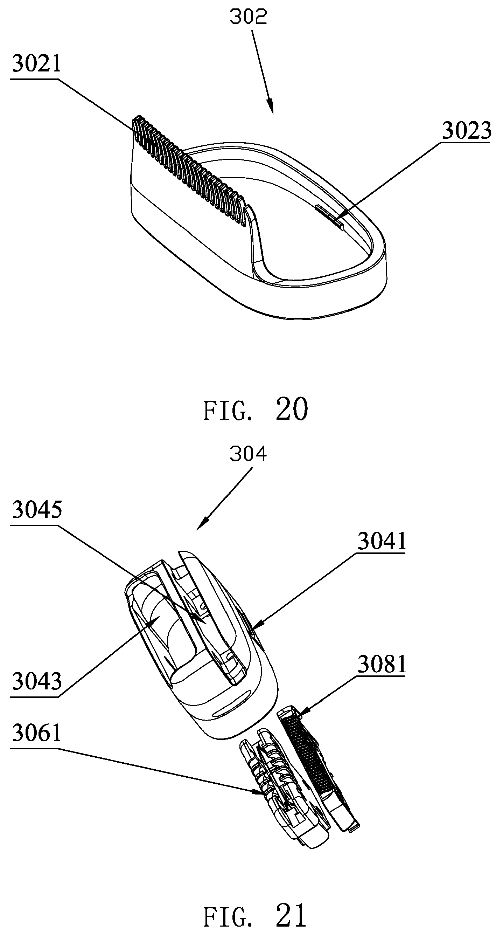

Referring to FIG. 20, the ring-shaped comb 302 includes a plurality of comb teeth 3021, uniformly space at nearly half of circumference of the ring-shaped comb 302. The comb teeth 3021 are used to comb the hair on the skin surface, for subsequently easy removal. The ring-shaped comb 302 further has a clip 3023 for position the ring-shaped comb 302 on the head 304.

Referring to FIG. 21, the head 304 is rectangular shaped, which has four arc-shaped smooth transitions at four corners. The head 304 has a heating groove 3043 for accommodating the heating element 306, and a cutting groove 3045 for accommodating the cutting element 308, arranged side by side in parallel. The heating groove 3043 is larger than a size of the cutting groove 3045. The head 304 further has a clip groove 3041, which is formed at an outer wall thereof. The clip groove 3041 is corresponding to the clip 3023 of the ring-shaped comb 302, for cooperating with the clip 3023 for fixing the ring-shaped comb 302 thereon.

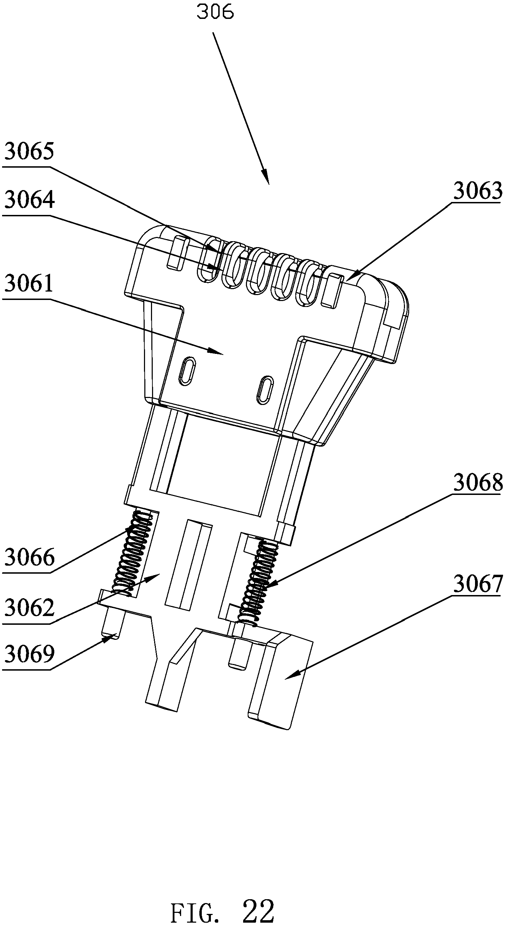

Referring to FIG. 22, the heating element 306 includes a heat generator 3061 at its front end, a slide rock 3062 connected with the heat generator 3061. The heat generator 3061 intermittently electrically connects with the PCB 309 through the slide rock 3062.

The heat generator 3061 includes a contact terminal 3063 and a heating wire 3065. The contact terminal 3063 is used to directly contact with the skin for flattening a surface of the skin. The contact terminal 3063 includes two pieces symmetrical contact pressing teeth 3064, defining a gap therebetween. The heating wire 3065 is accommodated in the gap. The position of the heating wire 3065 is lower than that of the contact pressing teeth 3064. That is, when the contact pressing teeth 3064 contacts with the skin at an initial stage, the heating wire 3065 doesn't contact with the skin. In use stage, after the two contact pressing teethes 3064 press the skin downwardly and flatten the hair thereon, the heating wire 3065 begin to burn out the hair. So that, the electrical hair removal device 300 can prevent scalding the skin. In other hand, the electrical hair removal device 300 utilizes the heating wire 3065 to burn out the hair and utilizes the heat to remove the hair root, and prohibits a hair re-growth speed. The heat generator 3061 has two conductive posts 3066 at one end, which transition fits at a top end of the slide rock 3062 through two conductive holes (not shown), respectively. Thus, the heat generator 3061 can be disassembled easily. Of course, as long as the heat generator 3061 can be easily disassembled, other fixing types also can be accepted, such as magnetic type, snap type, etc. The two conductive posts 3066 respectively electrically connected to two metal pieces (not shown), which connects to two terminals of the heating wire 3065, i.e. positive and negative polarities of the heating wire 3065. When the heat generator 3061 is fixed at the slide rock 3062, the two conductive posts 3066 are accommodated in the two conductive holes, which can electrically connect to a conductive element of the slide rock 3062.

The slide rock 3062 further includes two electrical shock heads 3069 and two springs 3068. The two conductive posts 3066 are positioned at a top end of the slide rock 3062 and the two electrical shock heads 3069 are positioned at a bottom end of the slide rock 3062, which are corresponding to each other. The two springs 3068 respectively disposed between the two electrical shock heads 3069 and the two conductive posts 3066. The two electrical shock heads 3069 plug into bottom ends of the two springs 3066, respectively. The two conductive posts 3066 plug into top ends of the two springs 3066, respectively. In an initial state, the two conductive posts 3066 don't contact to the two electrical shock heads 3069. In a usage state, the two conductive posts 3066 contact with the two electrical shock heads 3069, respectively, under an elastic deformation of the two springs 3068 by an external force. Thus, the power from the PCB 309 is provided to the heat generator 3061 through an electrical connection between the two conductive posts 3066 and the two electrical shock heads 3069. When the external force is reduced or does not exist, the two springs 3068 starts reset, and the two conductive posts 3066 separates from the two electrical shock heads 3069. Thus, power is disconnected to the heating head 3061.

The slide rock 3062 further includes a stop block 3067 at one side of the electrical shock heads 3069. When the heat generator 3061 is pressed on the skin surface, the heat generator 3061 and the slide rock 3062 slide toward an inside of the main body 307. In the moving process, the stop block 3067 on the slide rock 3062 triggers a photoelectric sensor, for turning on the power supply of the PCB 309, and heating the heating element 306. Thus, an electric hair removal mode is turned on. Conversely, when the hair removal is completed, the heat generator 3061 leaves from the skin surface. Under the elastic deformation of the springs 3068, the heat generator 3061 and the slide rock 3062 resets backwardly. The stop black 3067 on the slide rock 3062 is restored to its original state, which doesn't trigger the photoelectric sensor and turns off the power supply from the PCB 309. The heat generator 3061 stops heat. Thus, an automatically trigger can realize an automatic switch of the heat generator 3061, which ensures the safety and convenience of epilation.

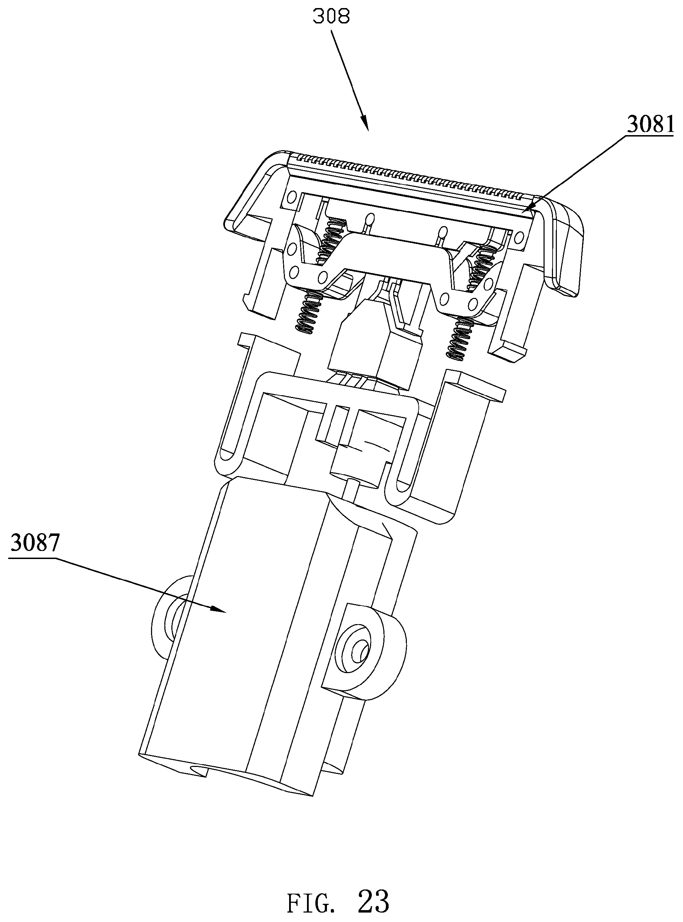

Referring to FIG. 23, the cutting element 308 is a controllable shaving components for cutting residual hair and short hair. The cutting element 308 includes a cutting head 3081 and a motor 3087. The cutting element 308 cooperates with the heating element 306 to remove the hair on the skin surface. In an operation sequence, the heating element 306 is used firstly, and the cutting element 308 is used sequentially. The heating element 306 and the cutting element 308 have a same height, for ensuring a same distance when the electrical hair removal device 300 is pressed on the skin surface. Thus, the press forces corresponding to the heating element 306 and the cutting element 308 are same in the operation process, which can ensure a comfortable using feeling. The motor 3087 provides a power to the cutting element 308 through the PCB 309.

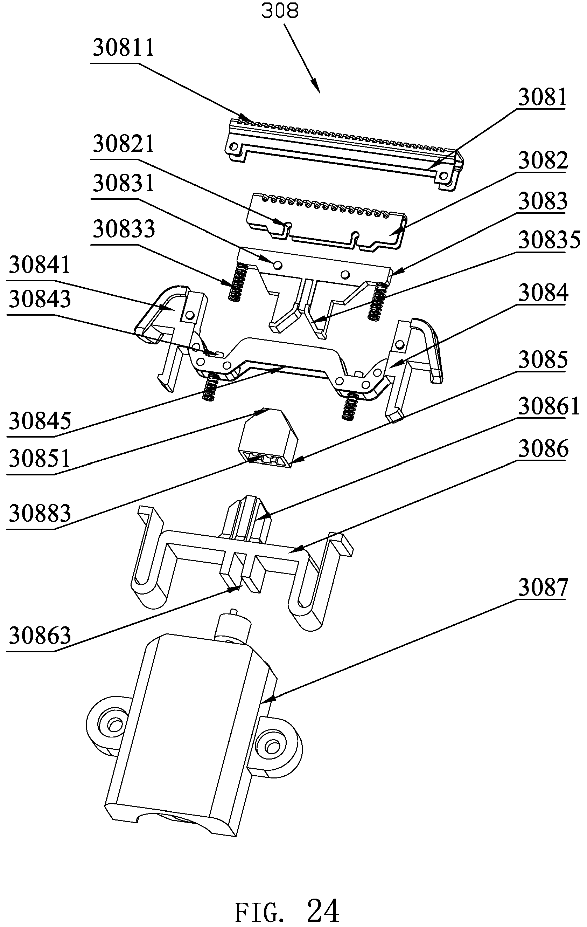

Referring to FIG. 24, the cutting element 308 has a cutting head 3081, a cutting blades 3082, a blade holder 3083, a connecting supporter 3084, a paddle sheath 3085, a paddle 3086 and a motor 3087, which are assembled in sequential. The cutting head 3081 includes a plurality of uniformly spaced blade gaps 30811 for receiving the removal needed hair. The cutting blades 3082 is accommodated in the cutting head 3081, which can move back and forth in the cutting head 3081 for cutting the hair received in the cutting head 3081. The cutting blade 3082 includes two engagement holes 30821 at its bottom, which respectively cooperate with two engagement columns 30831 of the blade holder 3083 thereon. When the cutting blade 3082 is disposed on the blade holder 3083, the two engagement columns 30831 are respectively locked in the two engagement holes 30821. Thus, the cutting blade 3082 are limited moving along one direction. The blade holder 3083 further has a toggle clamp 30835 at a bottom center, which is used to adapt the toggle plate 3086; and two holding springs 30833 at two bottom ends, for connecting to the connecting supporter 3084.

The connecting supporter 3084 fixing the cutting head 3081, the blade holder 3083 thereon, has a position state 30841, two spring posts 30843, and a toggle hole 30845. The cutting head 3081 is fixed on the position state 30841. The two spring posts 3084 connect with the two holding springs 30833, respectively, which let the blade holder 3083 and the cutting blades 3082 thereon automatically shrink and stretch along one direction. The toggle hole 30845 corresponds to the toggle clamp 30835, for the toggle plate 3086 passing through thereof and inserting into the toggle clamp 30835.

The toggle sheath 3085 includes a toggle head 30851 and an opening 30883 at bottom. The toggle head 30851 has a trapezoid section shape. When the toggle plate 3086 inserts into the opening 30883, the toggle head 30851 inserts into the toggle clamp 30835. Thus, the toggle plate 3086 can move with the cutting blade 3082 together, through the toggle sheath 3085 passing through the connecting supporter 3084.

The toggle plate 3086 has a guiding groove 30863 at a bottom end (not labeled), defining between two parallel disposed stop plates 30861.

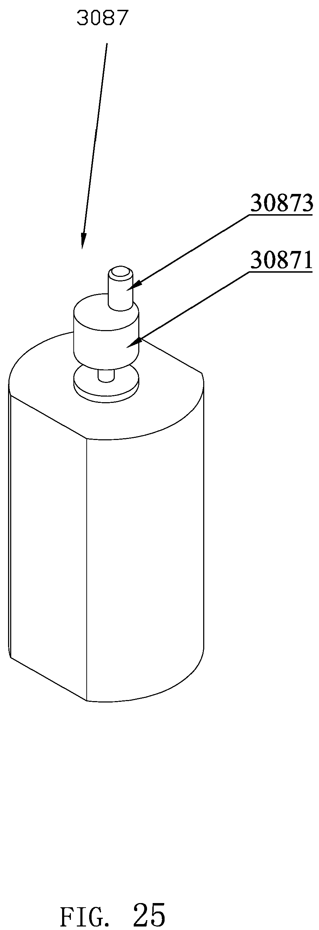

Referring to FIG. 25, the motor 3087 is a small power DC or AC motor, which has an output shaft 30871 at its top, and an eccentric column 30873 connected to the output shaft 30871. The eccentric column 30873 is inserted into the guiding groove 30863. Due to the eccentric column 30873 is not located in a center of the output shaft 30871, the eccentric column 30873 respectively touches with the two stop plates 30861, when the motor 3087 keeps to rotate along one direction. When a power is provided, the motor 3087 drives the toggle plate 3086 to horizontally swing. Then, the toggle sheath 3085, the blade holder 3083 and the cutting blade 3082 are driven to swing, and the cutting blade 3082 cuts off the hair inside the head 3081.

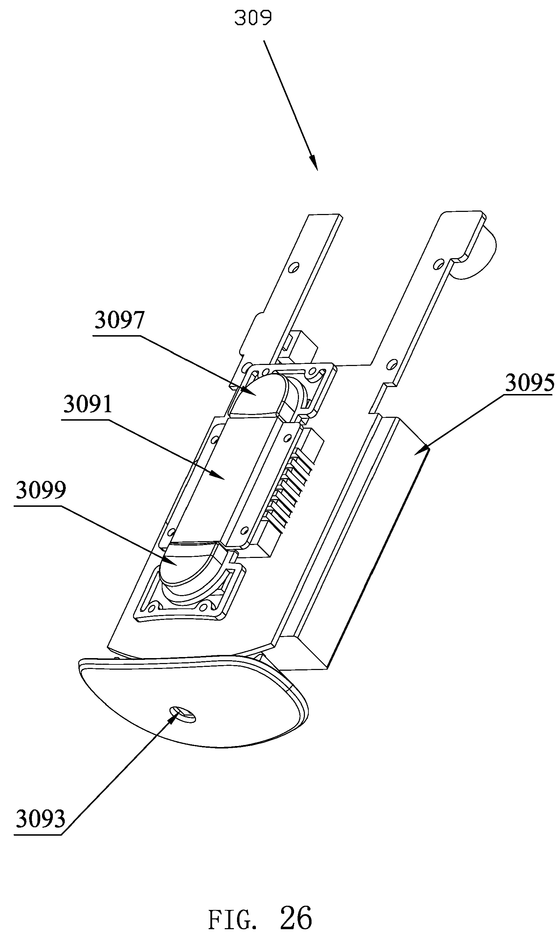

Referring to FIG. 26, the PCB 309 has a power supply 3095 at a bottom end, a keypad 3091 at a top end, and a charging hole 309 at a tail end, for charging the power supply 3095. The power supply 3095 electrically connects to the PCB 309 and is controlled to provide a needed voltage. The keypad 3091 has a mode key 3099 and a switch key 3097. In addition, the PCB 309 has a control system 200, which is similar to the control system 100 of the first embodiment.

An assembling process for the electrical hair removal device 300 has: 1^{st} step of assembling the heating element 306; 2^{nd} step of assembling the cutting element 308; 3^{rd} step of assembling the heating element 306 and the cutting element 308 on the ferrule 304; and 4^{th} step of fastening the top cover 303 and the bottom cover 305.

In the step of assembling the heating element 306, the heat generator 3061 transition fits at a top end of the slide rock 3062 , which two conductive posts 3066 pass through the two conductive holes of the slide rock 3062. In addition, the electrical shock head 3069 electrically connects to the PCB 309.

In the step of assembling the cutting element 308, firstly, the blade holder 3083 is positioned at the position state 30841 of the connecting supporter 3084, with the cutting blade 3082 fixed on the blade holder 3083. Here, the blade holder spring 30833 fits over the spring post 30843 of the connecting supporter 3084. Secondly, the toggle plate 3086 is inserted into the toggle sheath 3085 through the opening 30853, and the toggle sheath 3085 cooperates with the toggle clamp 30835 of the blade holder 3083. Thus, the toggle plate 3086, the toggle sheath 3085, the blade holder 3083 and the cutting blade 3082 can finish Synchronous lateral movement. Finally, the output shaft 30871 and the eccentric column 30873 of the motor 3087 are mounted between the two stop plates 30861. Thus, when the power is supplied, the motor 3087 can take the toggle plate 3086 to laterally sway. In use, the cutting head 3087 touches the skin of the user and keep unmovable, and the hair go into the cutting gap 30811. The motor 3087 drives the toggle plate 3086, the toggle sheath 3085, the blade holder 3083, and the cutting blade 3082 to periodically move laterally back and forth, for cutting the hair away.

In the step of assembling the heating element 306 and the cutting element 308 on the ferrule 304, the heating element 306 and the cutting element 308 are respectively mounted into the heating groove 3043 and the cutting groove 3045. The ring-shaped comb 302 is fixed around the peripheral of the ferrule 304, through the cooperation of the clip 3023 and the clip groove 3041.

Finally, in the step of fastening the top cover 303 and the bottom cover 305, the top cover 303 and the bottom cover 305 accommodate the heating element 306 and the cutting element 308 therein, with the ring-shaped comb 302 and ferrule 304 fixed thereon. The cap 301 is detachably fixed on the ring-shaped comb 302 and ferrule 304. In use, the cap 301 can be removed, and in no use, the cap 301 can protect the heating element 306 ad the cutting element 308.

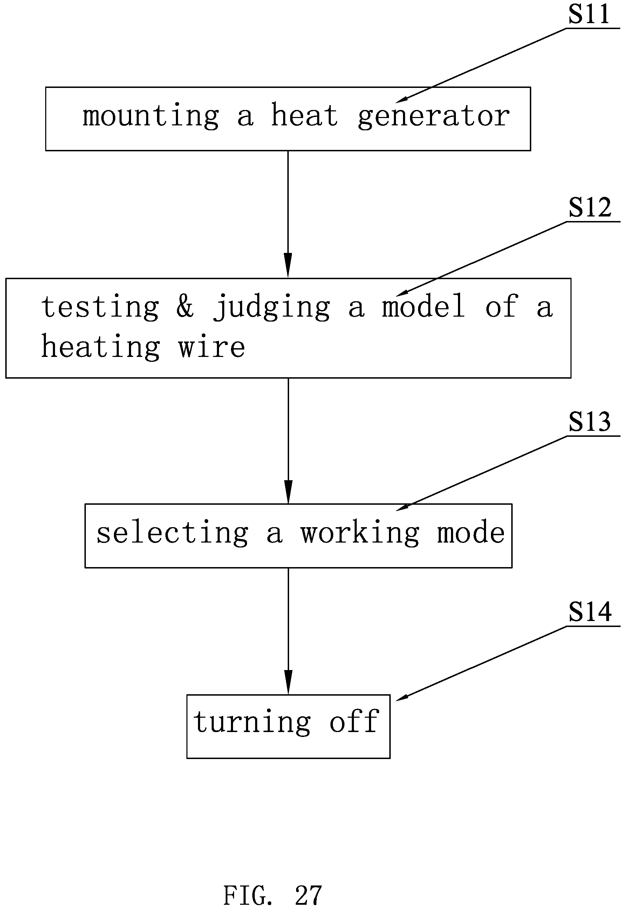

FIG. 27 is a flowchart of a control method for the electrical hair removal device 300 according to a second exemplary embodiment of the present invention.

In step S11, mounting the heat generator 3061, the user put the heat generator 3061 on the two conductive holes of the slide rock 3062. The user can choose different heating wires 3065, such as the longer heating wire, or the shorter heating wire. The heating temperature for the longer heating wire 3065 is 250° C - 450° C. The heating temperature for the shorter heating wire 3065 is 200° C - 400° C. When the hair is long and coarse, the user should choose the longer heating wire 3065. When the hair is short and fine, the user should choose the shorter heating wire 3065. In addition, the heating wire 3065 has a variety of types, is not limited to the above-described two types. The user can choose an appropriate heating wire, working at 200° C - 450 ° C temperature range.

In step S12, testing and judging a model of the heating wire 3065, the electrical hair removal device 300 turns on. The integrated amplifier circuit 205 begins to check the length of the heating wire 3065, and judges it for providing different driving voltage. For easily distinguishing different states, the electrical hair removal device 300 can add an indicating light. When the integrated amplifier circuit 205 doesn't detect the heating wire 3065, the electrical hair removal device 300 judges that the heating generator 3061 is not provided, or the heating wire 3065 is destroyed. Therefore, an alarm sound rings out. Thus, the user should turns off the power.

In step S13, selecting a working mode, the user presses the mode key 3099 for selecting different work modes according to the different needs, which electrically connecting to and is controlled by the control circuit 203. In this embodiment, three different work modes can be provided. For the first work mode, the heating element 306 and the cutting element 308 cooperate. For the second work mode, just the heating element 306 works. For the third work mode, just the cutting element 308 works.

In the first work mode, the heating element 306 works first, and then the cutting element 308 works sequentially. This work mode can utilize the heating element 306 to remove the longer hair firstly, and then utilize the cutting element 308 for removing the remaining stubble on the skin , which can realize a completely hair removal. When the user press the heat generator 3061 to the skin for a predetermined time, such as 1.5 seconds, the heating wire 3065 is heating and the motor 3087 works synchronous to drive the cutting element 308, until the heat generator 3061 leaves from the skin.

In the second work mode, only the heating element 306 is drove. This work mode is suitable for low depilation requirements.

In the third work mode, only the cutting element 308 is drove. Sometimes, the hair on the skin is very short, which looks unclean and unsmooth. But the heating element 306 isn't effective to the short hair. So, the cutting element 308 can finish the work perfectly.

In the above mentioned work modes, three different heating temperatures is further provided. If the heating wire 3065 is normally installed, and the control system 200 is on the standby state, the user presses the switch key 3097 long than two seconds, the switch circuit 207 converts the control system 200 to the usage state and the electrical hair removal device 300 works at the first gear. The user continues to press the switch key 3097 again, and the electrical hair removal device 300 works at the second gear. The user continues to press the switch key 3097 the third time, and the electrical hair removal device 300 works at the third gear. After that, the user press the switch key 3097 again, the electrical hair removal device 300 returns back to the first gear , for cycle operation. Finally, the user just needs to press the switch key 3097 long than three seconds, the electrical hair removal device 300 stops work.

In the hair removal process, the heat generator 3061 touches the skin, The contact terminal 3063 is used to directly contact with the skin for flattening a surface of the skin. The heat generator 3061 and the slide rock 3062 slide toward an inside of the main body 307. In the moving process, the stop block 3067 on the slide rock 3062 triggers the photoelectric sensor, for turning on the power supply of the PCB 309, and heating the heating element 306. In addition, when the heat generator 3061 is pressed down, an indicating lamp is lighting, for noticing the user that the electrical hair removal device 300 starts to work. Furthermore, a timelag, such as 2 seconds, is provided for user's preparation, which prevents scalding the skin by a hurried usage.

In an alternative solution, a microswitch can be used to replace the photoelectric sensor. When the heat generator 3061 and the slide rock 3062 slide toward an inside of the main body 307, the motion triggers the microswitch, for turning on the power supply of the PCB 309, and heating the heating element 306. When the contact terminal 3063 leaves the skin, the stop block 3067 and the heating element 306 begins to reset and trigger the microswitch again, which turns off the heating element 306 and the PCB 309.

In step S14, turning off, the user presses the switch key 3097, turning off the power supply 3095, the heat generator 3061 and the motor 3087 stop to work. In order to reflect the security and user-friendly design, the switch circuit is set on a timer (not shown) for deactivating the electrical hair removal device 300 at a set time. The electrical hair removal device 300 self-shutdowns after it works 15 minutes. This design can prevent the work time is too long resulting a lower cooling efficiency, and a potential safety hazards.

Compared with the prior art, the electrical hair removal device 100 allows an instant start upon use mode, i.e. only when the epilation starts, the electric heating wire 1063 automatically starts for hair removal, and as long as no hair removal needed or the electrical hair removal device 109 leaves the skin, the heating wire 1063 stops work. Therefore, its use is very safe and convenient. The electrical hair removal device 100 provides two ways to realize the instant start upon use mode. One is by the switch button 1091. The user only needs to press the switch button 1091 when depilation is needed. The heating wire 1063 is electrified for removing the hair through heat. When the hair removal is finished, the user only needs to release the switch button 1091, and the electrical hair removal device 100 returns to the standby state. This way utilizes a coordination and collaboration of human's hand and body to realize a human oriented use design. The other way uses a photoelectric sensor to control the heating wire 1063 for removing hair. As long as the contact terminal 1061 contacts with the skin surface, the photoelectric sensor is instantly triggered and let the heating wire 1063 to electrify for heating. This way utilizes an auto-sensing method to achieve the instant start upon use mode, which makes the use of the electrical hair removal device 100 more accurate and secure. In addition, the electrical hair removal device 100 provides multiple grade heating temperature ranges, the user can choose the right heating temperature range according to their own needs. For coarse hair, the user can choose a higher heating temperature grade. For fine hair, the user can choose a lower heating temperature grade. For users, this multiple heating selection settings become more practical and convenient. And the heating temperature is thermostat, which is achieved by providing the DC constant voltage power supply to the heating wire 1063. So, the control of the electrical hair removal device 100 becomes simpler. The depilatory area is heated evenly, and the depilatory efficiency is better. The electrical hair removal device 100 of the electrical hair removal device 100 utilizes a removable heat generator 1071. When the heat generator 1071 is needed to repair or replace, the user only needs to pull it down. This operation is easy to use and maintain. Furthermore, in the use state, the heating wire 1063 is not directly contact with the skin, so that the electrical hair removal device 100 can prevent the high temperature of the heating wire 1063 to burn the skin. In addition, the electrical hair removal device 100 utilizes the heating wire 1063 to burn out the hair and utilizes a high temperature to crimp hair roots thereof, the heating wire 1063 does not contact with the skin. Thus, a regeneration rate of the hair can be deferred.

In addition, the electrical hair removal device 300 further provides a new solution, which utilizes the heating element 306 and the cutting element 308 cooperation for removing the hair. The heating element 306 is used to remove the longer hair rapidly and painlessly, and the cutting element 308 is used to cut the shorter hair or the remaining hot-curled stubble immediately, after heat-removal process executed by the heating element 306. Because the hot-curled hair is very soft, the cutting element 308 can mechanically cut the hot-curled hair rapidly and painlessly. The cooperation of the heating element 306 and the cutting element 308 can completely remove the hair on the skin , no matter the longer hair or the shorter hair.

At the same time, all kinds of work modes are provided for different user's needs. In the first work mode, the heating element 306 works first, and then the cutting element 308 works sequentially. This work mode can utilize the heating element 306 to remove the longer hair firstly, and then utilize the cutting element 308 for removing the remaining hair on the skin , which can realize a completely hair removal. In the second work mode, only the heating element 306 is drove. This work mode is suitable for low depilation requirements. In the third work mode, only the cutting element 308 is drove. Sometimes, the hair on the skin is very short, which looks unclean and unsmooth. But the heating element 306 can't be effective to the short hair. So, the cutting element 308 can finish the work perfectly.

It is believed that the embodiments and their advantages will be understood from the foregoing description, and it will be apparent that various changes may be made thereto without departing from the spirit and scope of the invention or sacrificing all of its material advantages, the examples hereinbefore described merely being preferred or exemplary embodiments of the invention.

**List of Reference Numerals**

| | | | |
|---|---|---|---|
| electrical hair removal device | 100, 300 | cutting head | 3081 |
| main body | 107, 307 | comb teeth | 3021 |
| top cover | 103, 303 | clip | 3023 |
| bottom cover | 105, 305 | heating groove | 3043 |
| cap | 101, 301 | cutting groove | 3045 |
| top baffle | 1031, 3031 | slide rock | 3062 |
| bottom baffle | 1051 | contact terminal | 3063 |
| clips | 1021 | heating wire | 3065 |
| grooves | 1033 | Spring | 3068 |
| button hole | 1035 | stop block | 3067 |
| lamp holes | 1037 | cutting head | 3081 |
| heat generator | 1071, 3061 | motor | 3087 |
| supporting element | 1073 | a cutting blades | 3082 |
| fixing seat | 1075 | blade holder | 3083 |
| printed circuit board (PCB) | 1077, 309 | connecting supporter | 3084 |
| An ornamental frame | 1078 | paddle sheath | 3085 |
| button panel | 1079 | paddle | 3086 |
| contact terminal | 1061 | engagement column | 30831 |
| heating wire | 1063 | engagement hole | 30821 |
| lamp hole | 1065 | toggle clamp | 30835 |
| supporting body | 1066 | position state | 30841 |
| electrical shock head | 1068, 3069 | two spring post | 30843 |
| conductive post | 1069, 3066 | toggle hole | 30845 |
| illumination light | 1067 | holding spring | 30833 |
| battery cavity | 1084 | toggle head | 30851 |
| conductive terminals | 1081 | opening | 30883 |
| guiding wheel | 1083 | guiding groove | 30863 |
| battery | 1085 | output shaft | 30871 |
| switch button | 1091 | eccentric column | 30873 |
| blue start indicating light | 1092 | keypad | 3091 |
| red use indicating light | 1093 | charging hole | 309 |
| green charge indicating light | 1094 | mode key | 3099 |
| charging socket | 1095 | switch key | 3097 |
| head | 304 | | |
| ring-shaped comb | 302 | | |
| heating element | 306 | | |
| cutting element | 308 | | |
| power supply | 3095 | | |

## Claims

1. A control method for an electrical hair removal device, the electrical hair removal device comprising a heat generator having a heating wire formed thereon, comprising the steps of:
step S11: starting the electrical hair removal device and let it go into a standby state;
step S12: placing the heat generator on an hair removal needed area; and
step S13: electrifying the heating wire to a predetermined temperature, for realizing hair removal with a constant heating temperature.

2. The control method of claim 1, wherein the step S13 further includes the steps of providing a switch button, in use, pressing and holding the switch button, the heating wire continuously emitting heat, and releasing the switch button, the heating wire stopping emit heat.

3. The control method of claim 1, wherein the heating wire is supplied a DC power, and provides a constant heating power.

4. The control method of claim 1, wherein the heating wire delays to generate heat for 2-10 seconds, after it is electrical conducted.

5. The control method of any claim of claims 1, 2, 3, and 4, wherein, in step S13, the heating wire can work at 200° C -400 ° C heating temperature, or the heating wire can work at three different adjustable working states, the first working state being 200° C -300 ° C heating temperature; the second working state being 300° C -350 ° C heating temperature; and the third working state being 350° C -400 ° C heating temperature.

6. The control method of any claim of claims 1, 2, 3, and 4, wherein a gap between the heating wire and a skin surface is greater than 0 mm and less than 3 mm, in use.

7. The control method of claim 1, wherein step S13 further comprises providing a sensor, when the sensor is triggered, the heating wire is electrified for heating.

8. The control method of claim 7, wherein the sensor is triggered to electrify the heating wire, when the heat generator retracts backwardly by contacting with a skin surface, and the sensor stops work for disconnecting the heating wire, when the heat generator resets by an elastic deformation of a spring.

9. The control method of claim 1, wherein step S13 further comprises electrifying the heating wire to a predetermined temperature through placing the electrical hair removal device on the skin and moving the electrical hair removal device.

10. The control method of claim 1, wherein step S13 further comprises a timer or a calculagraph, and a step of automatically for deactivating the heating wire after a predetermined time.

11. The control method of claim 1, wherein in step S12, further comprising a cutting element, which cooperates with the heat generator for removing the unwanted hair, the heat generator being used to burn out the hair and utilizes the heat to removing the hair root, the cutting element being used to cut hair.

12. The control method of claim 11, wherein the heat generator is used to remove the longer hair, and the cutting element is used to remove the shorter hair.

13. The control method of claim 11, wherein in step S13, further comprises a switch key and a mode key, the switch key being used to switch the temperature scope of the heating wire, and the mode key being used to choose three different work modes, including the heat generator single working mode, the cutting element single working mode, and the heat generator and the cutting element cooperating mode.

14. An electrical hair removal device, comprising:
a main body, which comprises a heat generator having a heating wire thereon, a supporting element and a PCB ; and
a cover for accommodating the main body;
wherein the heating wire is electrically connected to the PCB, which is electrified and heated to a predetermined temperature, for realizing hair removal with a constant heating temperature.

15. The electrical hair removal device as claimed in claim 14, wherein the PCB comprises a conductive terminal, and the supporting element comprises an electrical shock head, the conductive terminal and the electrical shock head being electrically connected when the heat generator are poisoned on the skin.

16. The electrical hair removal device as claimed in claim 14, wherein a gyroscope is further provided therein, which electrifies the heating wire and drives the heating wire to heat up to the predetermined temperature, after the electrical hair removal device moves.

17. The electrical hair removal device as claimed in claim 14, wherein the electrical hair removal device further comprises a photoelectric sensor, When the electrical hair removal device touches the skin, which is triggered, and let the PCB to provide the power supply, and begin the hair removal; when the hair removal is completed, which the photoelectric sensor disconnects the power supply circuit of the heating wire and the heating wire stops emit heat; the heat generator and heating wire resets on the elastic deformation of the springs, through the photoelectric sensor disconnects the power supply circuit of the heating wire.

18. The electrical hair removal device as claimed in claim 15, further comprising a spring provided between the supporting element and the PCB, when the heating generator leaves away the skin surface, the electric shock head of the supporting element disconnects with the conductive terminal of the PCB by the elastic deformation of the spring.

19. The electrical hair removal device as claimed in any claim of claims 14, 15, 16, 17 and 18, wherein the heating wire can provide a heating temperature from 250 ° C to 450° C, or provide three different working states, that the first working state is 250° C -300 ° C heating temperature; the second working state is 300° C -400 ° C heating temperature; and the third working state is 400° C -450 ° C heating temperature.

20. The electrical hair removal device as claimed in claim 14, wherein the main body further comprises a cutting element, which cooperates with the heat generator for removing the unwanted hair, the heat generator is used firstly, and the cutting element is used sequentially.

21. The electrical hair removal device as claimed in claim 20, wherein the cutting element comprises a cutting head and a motor , the motor driving the cutting head to move back and forward.

22. The electrical hair removal device as claimed in claim 20, further comprising a comb, which comprises a plurality of comb teeth, being used to comb the hair on the skin surface, for subsequently easy removal.

23. The electrical hair removal device as claimed in claim 14, wherein the backwardly retraction of the heat generator can bring a photoelectric trigger by a photoelectric sensor, or bring an electrical connection for is supplying a DC power to the heating wire.
